# EUROPEAN PATENT APPLICATION

(11) **EP 4 166 573 A1**
(43) Date of publication of application: **19.04.2023**
(21) Application number: 21821084.7
(22) Date of filing: 10.06.2021
(51) Int. Cl.: C07K 19/00, A61K 38/28, C07K 14/62, A61P 5/50, C12N 15/11

(54) **INSULIN DEGLUDEC DERIVATIVE, PREPARATION METHOD THEREFOR, AND APPLICATION THEREOF**

(30) Priority: 10.06.2020 CN 202010524886
(71) Applicant: Ningbo Kunpeng Biotech Co., Ltd., Yuyao Ningbo, Zhejiang 315400 (CN)
(72) Inventor: YU, Ge, Ningbo, Zhejiang 315400 (CN); CHEN, Wei, Ningbo, Zhejiang 315400 (CN); LUO, Li, Ningbo, Zhejiang 315400 (CN)
(74) Representative: Lavoix
(86) International application number: PCT/CN2021/099493
(87) International publication number: WO 2021/249505

(57) **Abstract**

The present invention provides an insulin degludec derivative and a preparation method therefor. Specifically, the present invention provides a fusion protein comprising a green fluorescent protein folding unit and an insulin degludec precursor or an active fragment thereof. The fusion protein of the present invention has a significantly increased expression level, and the insulin degludec precursor protein in the fusion protein is folded correctly, and has biological activity. Moreover, the green fluorescent protein folding unit in the fusion protein of the present invention can be digested into small fragments by a protease, which have a great difference in molecular weight in comparison to a target protein, and are easy to separate. The present invention further provides a method for using the fusion protein to prepare insulin degludec and prepare an intermediate thereof.

## Description

### Technical field

The present invention relates to the field of biomedicine, in particular to an insulin degludec derivative and application thereof.

### Background

Diabetes is a major disease that threatens human health worldwide. In China, with the change of people's lifestyle and the acceleration of aging, the prevalence of diabetes is increasing rapidly. Acute and chronic complications of diabetes, especially the chronic complications, involve multiple organs, cause high disability and mortality rates, seriously affect the physical and mental health of patients, and bring heavy burdens to individuals, families and society.

Recombinant insulin degludec injection "Tresiba", developed by Novo Nordisk, Denmark, is a new long-acting insulin analogue. It was approved by the European Union in January 2013 for the treatment on patients with type I and type II diabetes. The structural feature of insulin degludec is that the εNH2 group on side chain of the lysine at position 29 of the B-chain of recombinant human insulin (in which the threonine at position 30 of B chain is removed) is conjugated with the modifier 16 carbon fatty diacid through an L-γ-Glu linker. Such design provides a unique mechanism to extend the acting time. Such insulin analogue has the advantages of long action time, low variability, and the ability to form a compound formulation with rapid-acting insulin. It forms a hexameric structure after subcutaneous injection, which can be used as a repository to slowly release insulin degludec monomers that can be slowly and continuously absorbed and utilized.

There are many domestic and foreign reports on the preparation of insulin degludec. Generally the insulin degludec main chain (backbone) is obtained through a genetic recombinant technology, and then the tBuO-Pal-Glu (OSu)-OtBu substance is connected by liquid phase synthesis method to obtain insulin degludec. Due to that the insulin degludec main chain is in an unprotected state, the side chain is easily connected to the N-terminal amino group during the connection process, thereby generating impurities, resulting in difficulty of purification and low yield, and the process is more complicated.

Therefore, those skilled in the art are committed to develop new, longer-acting insulin derivatives.

### Summary of the invention

The purpose of the present invention is to provide an insulin degludec derivative and application thereof.

In the first aspect of the present invention, it provides an insulin degludec precursor fusion protein having the structure as set forth in Formula III from N-terminus to the C-terminus:

A-FP-TEV-R-G (III)

wherein,
"-" represents a peptide bond;
A is absent or a leader peptide,
FP is a green fluorescent protein folding unit,
TEV is a first restriction site, and preferably is a restriction site of TEV enzyme;
R is arginine or lysine used for enzyme digestion;
G is an insulin degludec major chain or an active fragment thereof;
wherein the green fluorescent protein folding unit comprises 2 to 6 β-folding unit selected from the group consisting of:

| β-folding unit | Amino acid sequence |
|---|---|
| u1 | VPILVELDGDVNG (SEQ ID NO. 11) |
| u2 | HKFSVRGEGEGDAT (SEQ ID NO. 12) |
| u3 | KLTLKFICTT (SEQ ID NO. 13) |
| u4 | YVQERTISFKD (SEQ ID NO. 14) |
| u5 | TYKTRAEVKFEGD (SEQ ID NO. 15) |
| u6 | TLVNRIELKGIDF (SEQ ID NO. 16) |
| u7 | HNVYITADKQ (SEQ ID NO. 17) |
| u8 | GIKANFKIRHNVED (SEQ ID NO. 18) |
| u9 | VQLADHYQQNTPIG (SEQ ID NO. 19) |
| u10 | HYLSTQSVLSKD (SEQ ID NO. 20) |
| u11 | HMVLLEFVTAAGI (SEQ ID NO. 21). |

In another preferred embodiment, the green fluorescent protein folding unit is u2-u3, u4-u5, u1-u2-u3, u3-u4-u5 or u4-u5-u6.

In another preferred embodiment, the G is a Boc-modified insulin degludec precursor having the structure as set forth in Formula IV:

GB-X-GA (IV)

wherein,
"-" represents a peptide bond;
GB is the B chain of insulin degludec modified with Boc at position 29, whose amino acid sequence is set forth in SEQ ID NO. 2,
X is a linker peptide, and preferably the amino acid sequence of the linker peptide is R, RR, RRR or set forth in SEQ ID NO. 4-7; and
GA is the A chain of insulin degludec whose amino acid sequence is set forth in SEQ ID NO. 3.

In another preferred embodiment, the green fluorescent protein folding unit is u3-u4-u5.

In another preferred embodiment, the amino acid sequence of the green fluorescent protein folding unit is set forth in SEQ ID NO. 9.

In another preferred embodiment, the amino acid sequence of the insulin degludec precursor fusion protein is set forth in SEQ ID NO. 1.

In another preferred embodiment, the lysine at position 29 of the GB is an Nε-(tert-butoxycarbonyl)-lysine.

In another preferred embodiment, the B chain of insulin degludec comprised in the insulin degludec main chain does not comprise a side chain.

In another preferred embodiment, the GB does not comprise a side chain.

In the second aspect of the present invention, it provides an insulin degludec main chain fusion protein having the structure as set forth in Formula I from N-terminus to the C-terminus:

A-FP-TEV-R-D (I)

wherein,
"-" represents a peptide bond;
A is absent or a leader peptide,
FP is a green fluorescent protein folding unit;
TEV is a first restriction site, and preferably is a restriction site of TEV enzyme (as set forth in sequence ENLYFQG, SEQ ID NO. 10);
R is arginine or lysine used for enzyme digestion;
D is a Boc-modified insulin degludec main chain having the structure as set forth in Formula II: 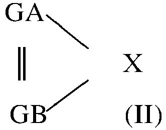 wherein,
"∥" represents a disulfide bond;
GA is the A chain of insulin degludec whose amino acid sequence is set forth in SEQ ID NO. 3,
X is a linker peptide;
GB is the B chain of insulin degludec modified with Boc at position 29, whose amino acid sequence is set forth in SEQ ID NO. 2;
wherein the green fluorescent protein folding unit comprises 2 to 6 β-folding unit selected from the group consisting of:

| β-folding unit | Amino acid sequence |
|---|---|
| u1 | VPILVELDGDVNG (SEQ ID NO. 11) |
| u2 | HKFSVRGEGEGDAT (SEQ ID NO. 12) |
| u3 | KLTLKFICTT (SEQ ID NO. 13) |
| u4 | YVQERTISFKD (SEQ ID NO. 14) |
| u5 | TYKTRAEVKFEGD (SEQ ID NO. 15) |
| u6 | TLVNRIELKGIDF (SEQ ID NO. 16) |
| u7 | HNVYITADKQ (SEQ ID NO. 17) |
| u8 | GIKANFKIRHNVED (SEQ ID NO. 18) |
| u9 | VQLADHYQQNTPIG (SEQ ID NO. 19) |
| u10 | HYLSTQSVLSKD (SEQ ID NO. 20) |
| u11 | HMVLLEFVTAAGI (SEQ ID NO. 21). |

In another preferred embodiment, the R is used for trypsin digestion and carboxypeptidase digestion.

In another preferred embodiment, the C-terminus of the B chain of insulin degludec is connected to the N-terminus of the A chain of insulin degludec through a linker peptide.

In another preferred embodiment, the amino acid sequence of the linker peptide is R, RR, RRR or set forth in SEQ ID NO. 4-7 (RRGSKR, RRAAKR, RRYPGDVKR or RREAEDLQVGQVELGGGPGAGSLQPLALEGSLQKR).

In another preferred embodiment, interchain disulfide bonds are formed between position 7 of B chain and position 7 of A chain (A7-B7), and between position 19 of B chain and position 20 of A chain (A20-B19) in the insulin degludec.

In another preferred embodiment, intrachain disulfide bond is formed between position 6 of A chain and position 11 of A chain (A6-A11) in the insulin degludec.

In another preferred embodiment, the green fluorescent protein folding unit is u2-u3, u4-u5, u1-u2-u3, u3-u4-u5 or u4-u5-u6.

In another preferred embodiment, the green fluorescent protein folding unit is u3-u4-u5.

In another preferred embodiment, the Boc-modified insulin degludec main chain comprises an A chain and a B chain, and the A chain and B chain are connected with an interchain disulfide bond, and preferably with two interchain disulfide bond.

In another preferred embodiment, the A chain comprises an intrachain disulfide bond.

In another preferred embodiment, the sequence of the leader peptide is set forth in SEQ ID NO. 8.

In the third aspect of the present invention, it provides a Boc-modified insulin degludec precursor having the structure as set forth in Formula IV:

GB-X-GA (IV)

wherein,
"-" represents a peptide bond;
GB is the B chain of insulin degludec modified with Boc at position 29, whose amino acid sequence is set forth in SEQ ID NO. 2,
X is a linker peptide, and preferably the amino acid sequence of the linker peptide is R, RR, RRR or set forth in SEQ ID NO. 4-7; and
GA is the A chain of insulin degludec whose amino acid sequence is set forth in SEQ ID NO. 3.

In the fourth aspect of the present invention, it provides a Boc-modified insulin degludec main chain having the structure as set forth in Formula II: 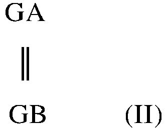 wherein,
" ∥" represents a disulfide bond;
GA is the A chain of insulin degludec whose amino acid sequence is set forth in SEQ ID NO. 3, and
GB is the B chain of insulin degludec whose amino acid sequence is set forth in SEQ ID NO. 2, and the position 29 of the B chain is an Nε-(tert-butoxycarbonyl)-lysine.

In the fifth aspect of the present invention, it provides a Boc and Fmoc-modified insulin degludec main chain having the structure as set forth in Formula II: 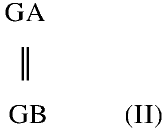 wherein,
"∥" represents a disulfide bond;
GA is the A chain of insulin degludec whose amino acid sequence is set forth in SEQ ID NO. 3,
GB is the B chain of insulin degludec whose amino acid sequence is set forth in SEQ ID NO. 2, and the position 29 of the B chain is an Nε-(tert-butoxycarbonyl)-lysine.
and the N-terminus of both A chain and B chain are modified with Fmoc.

In another preferred embodiment, the Fmoc is a fluorenylmethoxycarbonyl.

In the sixth aspect of the present invention, it provides an Fmoc-modified insulin degludec main chain having the structure as set forth in Formula II: 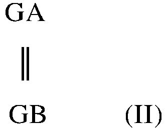 wherein,
"∥" represents a disulfide bond;
GA is the A chain of insulin degludec whose amino acid sequence is set forth in SEQ ID NO. 3,
GB is the B chain of insulin degludec, whose amino acid sequence is set forth in SEQ ID NO. 2,
and the N-terminus of both A chain and B chain are modified with Fmoc.

In another preferred embodiment, the Fmoc is a fluorenylmethoxycarbonyl.

In the seventh aspect of the present invention, it provides a method for preparing an insulin degludec, comprising the steps:
(A) using a recombinant bacteria to ferment, to prepare an insulin degludec precursor fusion protein, and
(B) using the insulin degludec precursor fusion protein to prepare insulin degludec,
   wherein the insulin degludec precursor fusion protein is as described in the first aspect of the present invention.

In another preferred embodiment, the step (B) further comprises the steps:
(ia) isolating and obtaining inclusion bodies of the insulin degludec precursor fusion protein from the fermentation broth of the recombinant bacteria, renaturing the inclusion bodies and obtaining the insulin degludec main chain fusion protein;
(ib) digesting the insulin degludec main chain fusion protein with enzyme, thereby obtaining a Boc-modified insulin degludec main chain;
(ii) modifying the Boc-modified insulin degludec main chain with Fmoc, thereby obtaining the Fmoc and Boc-modified insulin degludec main chain;
(iii) removing the Boc from the Fmoc and Boc-modified insulin degludec main chain, thereby obtaining a Boc-removed insulin degludec main chain;
(iv) reacting the Boc-removed insulin degludec main chain with an insulin degludec side chain, thereby obtaining a Fmoc-modified insulin degludec; and
(v) removing the Fmoc from the Fmoc-modified insulin degludec and the OtBu from the side chain, thereby obtaining the insulin degludec.

In another preferred embodiment, in step (ib), trypsin and carboxypeptidase B are used for the digestion.

In another preferred embodiment, the Boc-modified insulin degludec main chain is as described in the fourth aspect of the present invention.

In another preferred embodiment, the Fmoc modification is a modification on the N-terminus of the B chain and A chain of insulin degludec.

In another preferred embodiment, the insulin degludec side chain is as follow:

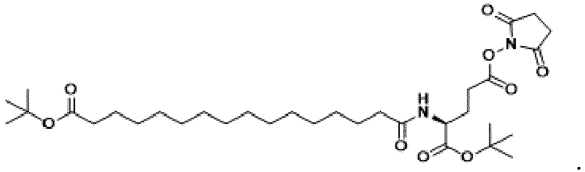

In another preferred embodiment, in the step (ii), Fmoc-Osu, DIPEA (N, N-diisopropylethylamine) and DMF (N, N-dimethylformamide) are added to carry out Fmoc modification.

In another preferred embodiment, the molar ratio of the added Fmoc-Osu, DIPEA and Boc-modified insulin degludec main chain is (3-6): (10-14): (0.8-1.2), and preferably (3.5-5.5): (11-13): (0.8-1.2).

In another preferred embodiment, it further comprises a step of purification of the obtained Fmoc and Boc-modified insulin degludec main chain between steps (ii) and (iii), preferably by using a mixture of methyl tert-butyl ether/petroleum ether.

In another preferred embodiment, in the step (iii) it further comprises the steps:
(a) adding a Compound 2 to pre-cooled TFA (trifluoroacetic acid) solution at 0±5°C, stirring to remove Boc and obtaining a Boc-removed product;
(b) purifying the Boc-removed product, preferably by using a mixture of methyl tert-butyl ether/petroleum ether, thereby obtaining a solid Boc-removed product, i.e. a Boc-removed insulin degludec main chain.

In another preferred embodiment, in the step (iv), the reaction is carried out in the presence of DIPEA at room temperature.

In another preferred embodiment, in the step (iv), the reaction is carried out in DMF.

In another preferred embodiment, the molar ratio of the Boc-removed insulin degludec main chain, insulin degludec side chain and DIPEA is (0.8-1.2): (2-5): (4-10), and preferably 1:2.5:10.

In another preferred embodiment, in the step (v), DMF solution containing piperidine is added to remove Fmoc, thereby the insulin degludec is obtained.

In another preferred embodiment, in the step (v), it further comprises a step of purification of the obtained insulin degludec.

In another preferred embodiment, the Boc-modified insulin degludec main chain is produced by using genetic recombination technique.

In another preferred embodiment, it further comprises two purification steps after step (ib).

In another preferred embodiment, in the step (ib), the mass ratio of trypsin to insulin degludec precursor fusion protein is 1:3000-1:10000.

In another preferred embodiment, in the step (ib), the mass ratio of carboxypeptidase to insulin degludec precursor fusion protein is 1:5000-1:15000.

In another preferred embodiment, the recombinant bacteria comprise or are integrated with an expression cassette expressing the insulin degludec precursor fusion protein.

In another preferred embodiment, the method is as follows:

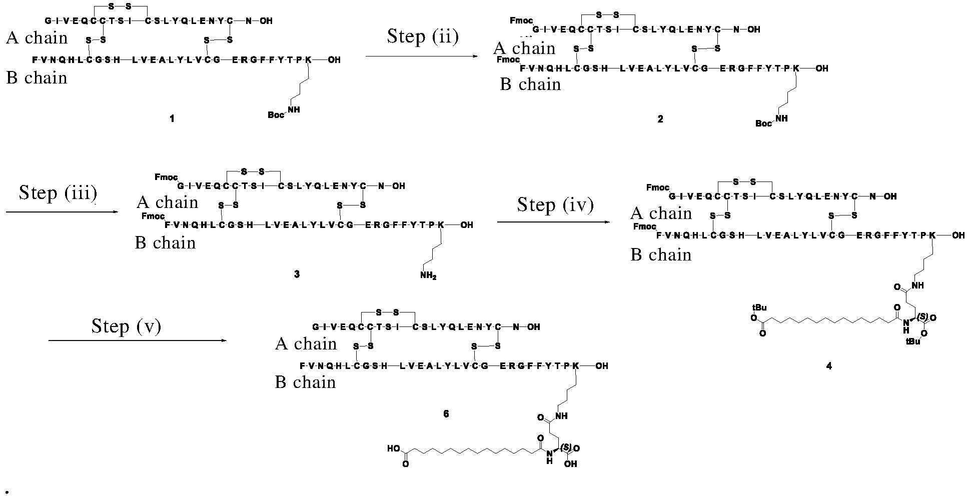

In another preferred embodiment, the method comprises the steps:
(i) providing the insulin degludec main chain fusion protein of the second aspect of the present invention, enzyme digesting to obtain Compound 1;
(ii) modifying the Compound 1 with Fmoc, thereby obtaining Compound 2;
(iii) removing Boc from the Compound 2, thereby obtaining Compound 3;
(iv) reacting Compound 3 with the insulin degludec side chain, thereby obtaining Compound 4; and
(v) removing the Fmoc from Compound 4 and the OtBu from the side chain thereof, thereby obtaining the insulin degludec as shown in Compound 6.

In an eighth aspect of the present invention, it provides an insulin degludec formulation produced by using the method of the seventh aspect of the present invention.

In another preferred embodiment, the produced insulin degludec has bioactivity.

In the ninth aspect of the present invention, it provides an isolated polynucleotide encoding the insulin degludec precursor fusion protein of the first aspect of the present invention, the insulin degludec main chain fusion protein of the sexond aspect of the present invention, the insulin degludec precursor of the third aspect of the present invention, or the insulin degludec main chain of the fourth, fifth, or sixth aspect of the present invention.

In the tenth aspect of the present invention, it provides a vector comprising the polynucleotide of the ninth aspect of the present invention.

In another preferred embodiment, the vector is selected from the group consisting of DNA, RNA, plasmids, lentiviral vectors, adenoviral vectors, retroviral vectors, transposons, and a combination thereof.

In the eleventh aspect of the present invention, it provides a host cell comprising the vector of the tenth aspect of the present invention or in which the chromosome is integrated with exogenous polynucleotide of the ninth aspect of the present invention.

In another preferred embodiment, the host cell is Escherichia coli, Bacillus subtilis, a yeast cell, an insect cell, a mammalian cell or a combination thereof.

In the twelfth aspect of the present invention, it provides a formulation or pharmaceutical composition comprising the insulin degludec precursor fusion protein of the first aspect of the present invention, the insulin degludec main chain fusion protein of the second aspect of the present invention, the insulin degludec precursor of the third aspect of the present invention, or the insulin degludec main chain of the fourth, fifth, or sixth aspect of the present invention, and a physiologically acceptable carrier.

In the thirteenth aspect of the present invention, it provides an Fmoc-modified insulin degludec main chain having the structure as set forth in Formula II': 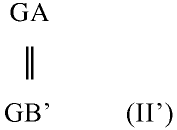 wherein,
"∥" represents a disulfide bond;
GA is the A chain of insulin degludec whose amino acid sequence is set forth in SEQ ID NO. 3,
GB' is the B chain of insulin degludec whose amino acid sequence is set forth in SEQ ID NO. 2, and the lysine at position 29 of the B chain is connected to a side chain of insulin degludec;
and the N-terminus of both A chain and B chain are modified with Fmoc.

In another preferred embodiment, the insulin degludec side chain is as follow:

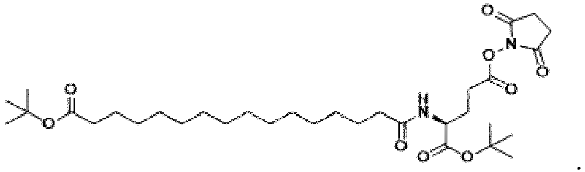

In another preferred embodiment, the insulin degludec side chain is tBuO-Pal-Glu (OSu)-OtBu.

### Description of the Figures

Figure 1 shows a map of the plasmid pBAD-FP-TEV-R- Insulin (DesB30, Lys²⁹Boc).
Figure 2 shows a map of the plasmid pEvol-pylRs-pylT.
Figure 3 shows the SDS-PAGE electrophoresis figure of the Boc-insulin degludec main chain fusion protein after renaturation of the inclusion body.
Figure 4 shows the HPLC detection spectrogram of the reversed-phase purified collection of Boc-insulin degludec main chain.
Figure 5 shows the HPLC detection spectrogram of the purified final product of insulin degludec of the present invention.

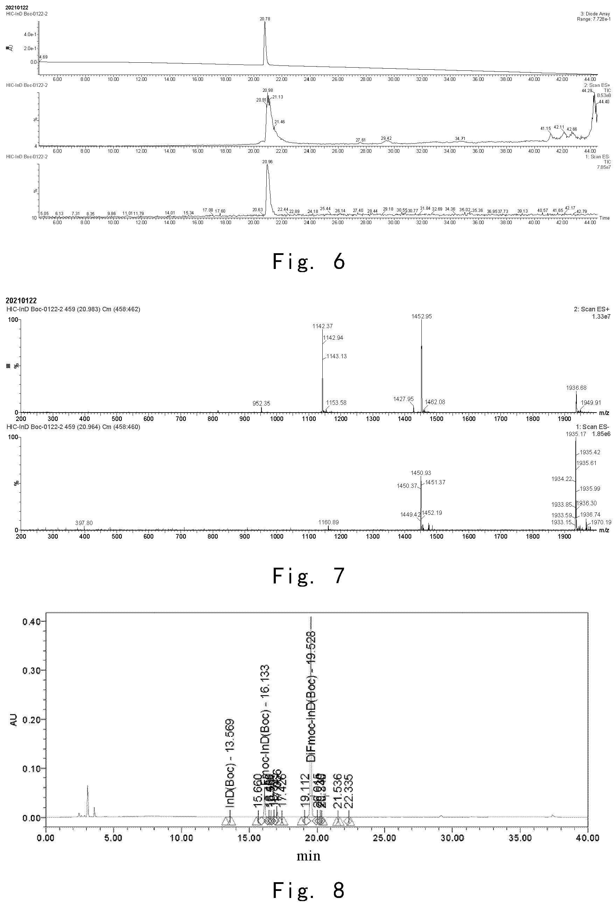

Figure 6 shows the HPLC detection spectrogram of the Boc-insulin degludec main chain of Example 4 (Compound 1).
Figure 7 shows the LCMS detection spectrogram of the Boc-insulin degludec main chain of Example 4 (Compound 1), wherein the measured molecular weight is 5807.8 and the theoretical molecular weight is 5806.6.
Figure 8 shows the HPLC detection spectrogram of the Fmoc and Boc-protected main chain of Example 5 (Compound 2), wherein the purity of the HPLC spectrogram is 86.26%.

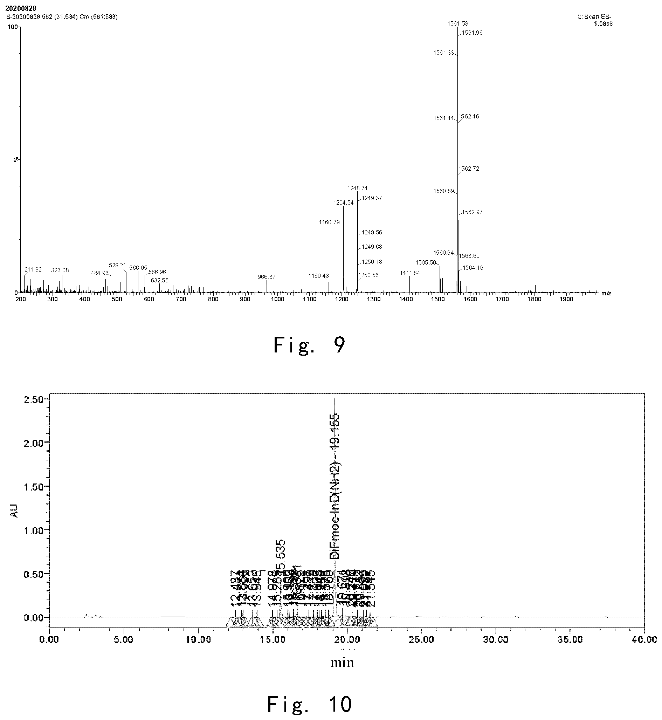

Figure 9 shows the LCMS detection spectrogram of the Fmoc and Boc-protected main chain of Example 5 (Compound 2), wherein the measured molecular weight is 6150.3 and the theoretical molecular weight is 6251.1.
Figure 10 shows the HPLC detection spectrogram of the Fmoc-protected insulin degludec main chain of Example 5 (Compound 3), wherein the purity of the HPLC spectrogram is 83.02%.

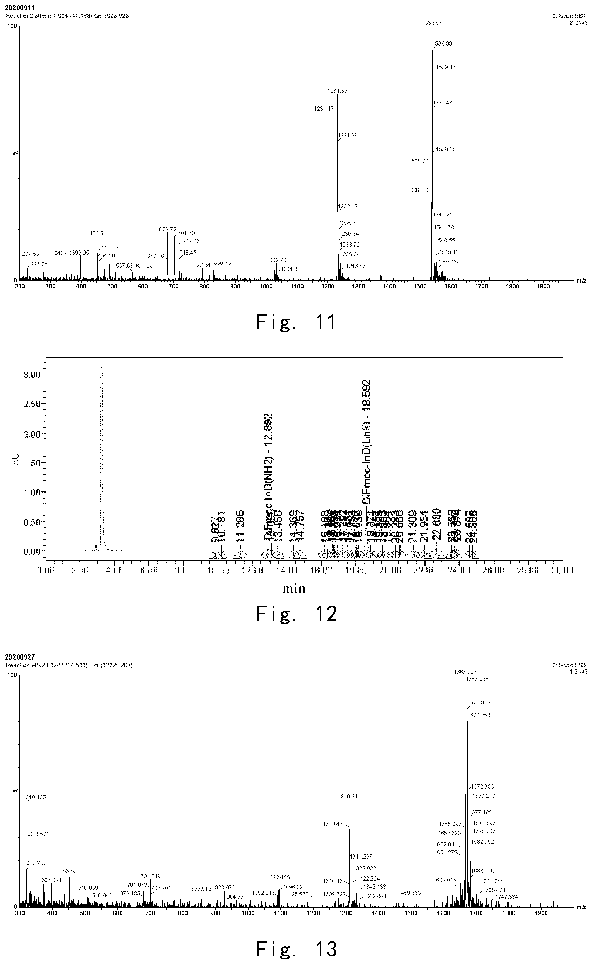

Figure 11 shows the LCMS detection spectrogram of the Fmoc-protected insulin degludec main chain of Example 5 (Compound 3), wherein the measured molecular weight is 6150.7 and the theoretical molecular weight is 6251.1.
Figure 12 shows the HPLC detection spectrogram of the insulin degludec with Fmoc and OtBu on the side chain of Example 5 (Compound 4), wherein the purity of the HPLC spectrogram is 66.64%.
Figure 13 shows the LCMS detection spectrogram of the insulin degludec with Fmoc and OtBu on the side chain of Example 5 (Compound 4), wherein the measured molecular weight is 6660.0 and the theoretical molecular weight is 6660.8.

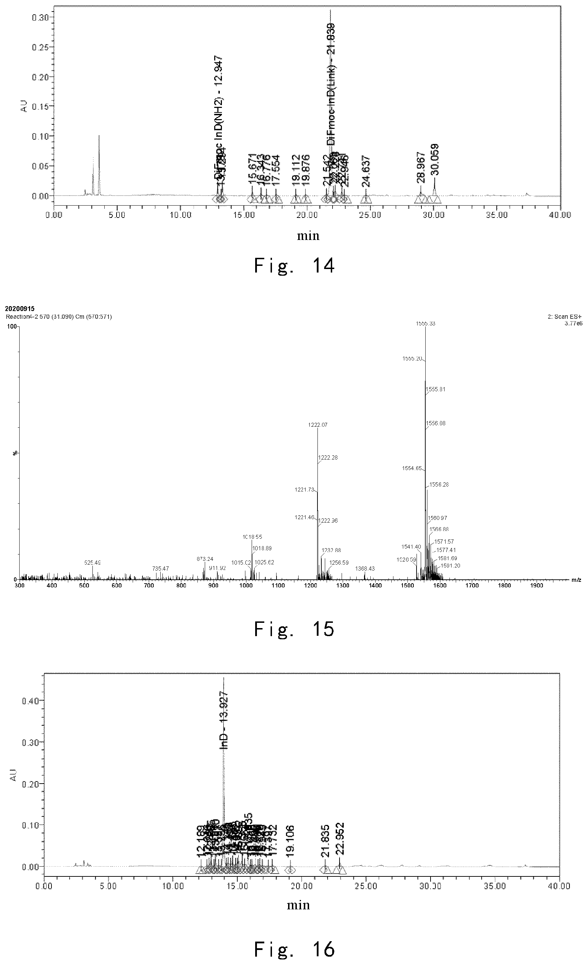

Figure 14 shows the HPLC detection spectrogram of the insulin degludec with OtBu on the side chain of Example 5 (Compound 5), wherein the purity of the HPLC spectrogram is 74.37%.
Figure 15 shows the LCMS detection spectrogram of the insulin degludec with OtBu on the side chain of Example 5 (Compound 5), wherein the measured molecular weight is 6217.3 and the theoretical molecular weight is 6216.3.
Figure 16 shows the HPLC detection spectrogram of the insulin degludec of Example 5 (Compound 6), wherein the purity of the HPLC spectrogram is 63.12%.

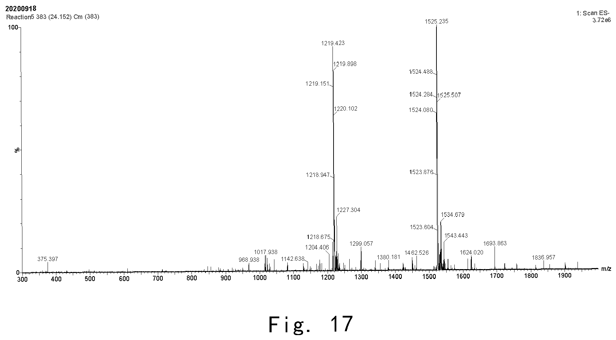

Figure 17 shows the LCMS detection spectrogram of the insulin degludec of Example 5 (Compound 6), wherein the measured molecular weight is 6104.0 and the theoretical molecular weight is 6104.9.

### DETAILED DESCRIPTION

After extensive and intensive research, the inventors have discovered an insulin delgludec derivative and preparation method therefor. Specifically, the present invention provides a fusion protein comprising a green fluorescent protein folding unit and an insulin degludec precursor or an active fragment thereof. The fusion protein of the present invention has a significantly increased expression level, and the insulin degludec precursor protein in the fusion protein is folded correctly. Moreover, the green fluorescent protein folding unit in the fusion protein of the present invention can be digested into small fragments by a protease, which have a great difference in molecular weight in comparison to the target protein, and are easy to separate. The present invention further provides a method for using the fusion protein to prepare insulin degludec and prepare an intermediate thereof. On this basis, the present invention has been completed.

### Insulin degludec

Insulin degludec is a new long-acting insulin analogue. It was approved by the European Union in January 2013 for the treatment on patients with type I and type II diabetes. The structural feature of insulin degludec is that the ε-NH2 group on side chain of the lysine at position 29 of the B-chain of recombinant human insulin (in which the threonine at position 30 of B chain is removed) is conjugated with the modifier 16 carbon fatty diacid through an L-γ-Glu linker. Such design provides a unique mechanism to extend the acting time. Such insulin analogue has the advantages of long action time, low variability, and the ability to form a compound formulation with rapid-acting insulin. It forms a hexameric structure after subcutaneous injection, which can be used as a repository to slowly release insulin degludec monomers that can be slowly and continuously absorbed and utilized.

### Construction of the plasmid expressing insulin degludec

The FP-TEV-R Insulin (DesB30, Lys29Boc) fragment which contains the target gene was synthesized, of which the two ends had the recognition sites of restriction endonucleases Nco I and Xho I. The codon of this sequence was optimized and can achieve high level expression of functional protein in *E. coli.* After expression, the restriction endonucleases Nco I and Xho I were used to cut the expression vector "pBAD/His A (KanaR)" and the plasmid containing the target gene "FP-TEV-R-Insulin (DesB30, Lys²⁹Boc)". The digested products were separated by agarose electrophoresis, and then extracted by agarose gel DNA recovery kit. Finally, the two DNA fragments were connected by T4 DNA ligase. The connected product was chemically transformed into *E. coli* Top10 cells, and the transformed cells were cultured in LB agar medium (10 g/L yeast peptone, 5 g/L yeast extract, 10 g/L NaCl, 1.5% agar) containing 50 µ g/mL kanamycin overnight. 3 live colonies were picked and cultured in 5mL liquid LB medium (10g/L yeast peptone, 5g/L yeast extract and 10g/L NaCl) containing 50µg/mL kanamycin overnight, and the plasmid was extracted by using small amount plasmid extraction kit. Then, the extracted plasmid was sequenced using the sequencing oligonucleotide primer 5 '- ATGCCATAGCATTTTTATCC-3' to confirm correct insertion. The finally obtained plasmid was named as "pBAD-FP-TEV-R-Insulin (DesB30, Lys²⁹Boc)".

### Fusion protein

By using the green fluorescent protein folding unit, the invention constructs two fusion proteins, namely the insulin degludec precursor fusion protein containing single chain insulin degludec according to the first aspect of the invention and the insulin degludec main chain fusion protein containing double chain insulin degludec according to the second aspect of the invention. In fact, the protection scope of the two fusion proteins of the present invention may overlap partially. For example, the C-terminus of the B chain of the double chain insulin degludec contained in the fusion protein can be connected to the N-terminus of the A chain through a linker peptide, which can also be considered as a single chain containing an intrachain disulfide bond.

The green fluorescent protein folding unit contained in the fusion protein of the present invention comprises 2 to 6, preferably 2 to 3, β-folding unit selected from the group consisting of:

| | Amino acid sequence |
|---|---|
| u1 | VPILVELDGDVNG (SEQ ID NO. 11) |
| u2 | HKFSVRGEGEGDAT (SEQ ID NO. 12) |
| u3 | KLTLKFICTT (SEQ ID NO. 13) |
| u4 | YVQERTISFKD (SEQ ID NO. 14) |
| u5 | TYKTRAEVKFEGD (SEQ ID NO. 15) |
| u6 | TLVNRIELKGIDF (SEQ ID NO. 16) |
| u7 | HNVYITADKQ (SEQ ID NO. 17) |
| u8 | GIKANFKIRHNVED (SEQ ID NO. 18) |
| u9 | VQLADHYQQNTPIG (SEQ ID NO. 19) |
| u10 | HYLSTQSVLSKD (SEQ ID NO. 20) |
| u11 | HMVLLEFVTAAGI (SEQ ID NO. 21) . |

In another preferred embodiment, the green fluorescent protein folding unit FP can be selected from the group consisting of: u8, u9, u2-u3, u4-u5, u8-u9, u1-u2-u3, u2-u3-u4, u3-u4-u5, u5-u6-u7, u8-u9-u10, u9-u10-u11, u3-u5-u7, u3-u4-u6, u4-u7-u10, u6-u8-u10, u1-u2-u3-u4, u2-u3-u4-u5, u3-u4-u3-u4, u3-u5-u7-u9, u5-u6-u7-u8, u1-u3-u7-u9, u2-u2-u7-u8, u7-u2-u5-u11, u3-u4-u7-u10, u1-I-u2, u1-I-u5, u2-I-u4, u3-I-u8, u5-I-u6, and u10-I-u11.

In another preferred embodiment, the green fluorescent protein folding unit is u3-u4-u5.

In another preferred embodiment, the fusion protein of the present invention has the structure as shown in the following formula:

A-FP-TEV-R-G

wherein,
"-" represents a peptide bond;
A is absent or a leader peptide,
FP is a green fluorescent protein folding unit,
TEV is a restriction site, and preferably is a restriction site of TEV enzyme (whose sequence is ENLYFQG, SEQ ID NO. 4);
R is a restriction site;
G is a Boc-modified insulin degludec precursor and G has the structure as set forth in the following formula:

   (BlF~B29Boc-K)- X-(A1G~A21N)

   wherein,
X is a linker peptide, and preferably the amino acid sequence of the linker peptide is R, RR, RRR or set forth in SEQ ID NO. 4-7 (RRGSKR, RRAAKR, RRYPGDVKR or RREAEDLQVGQVELGGGPGAGSLQPLALEGSLQKR).

As used herein, the term "fusion protein" also includes variant forms having the above-mentioned activities. These variant forms include (but are not limited to): 1-3 (usually 1-2, more preferably 1) amino acid deletions, insertions and/or substitutions, and one or several (usually 3 or less, preferably 2 or less, more preferably 1 or less) amino acids added or deleted at the C-terminal and/or N-terminal. For example, in this field, when substituted with amino acids with close or similar properties, the function of the protein is usually not changed. For another example, adding or deleting one or several amino acids at the C-terminus and/or N-terminus usually does not change the structure and function of the protein. In addition, the term also includes the polypeptide of the present invention in monomeric and multimeric forms. The term also includes linear and non-linear polypeptides (such as cyclic peptides).

The present invention also includes active fragments, derivatives and analogs of the above-mentioned fusion protein. As used herein, the terms "fragment", "derivative" and "analog" refer to a polypeptide that substantially retains the function or activity of the fusion protein of the present invention. The polypeptide fragments, derivatives or analogs of the present invention can be (i) a polypeptide in which one or more conservative or non-conservative amino acid residues (preferably conservative amino acid residues) are substituted, or (ii) a polypeptide with a substitution group in one or more amino acid residues, or (iii) a polypeptide formed by fusion of a polypeptide with another compound (such as a compound that prolongs the half-life of polypeptide, such as polyethylene glycol), or (iv) the polypeptide formed by fusion of additional amino acid sequence to this polypeptide sequence (fusion protein formed by fusion with a tag sequence such as leader sequence, secretory sequence or 6His). According to the teachings herein, these fragments, derivatives and analogs fall within the scope well known to those skilled in the art.

A preferred type of active derivative means that compared with the amino acid sequence of the present invention, at most 3, preferably at most 2, and more preferably at most 1 amino acid are replaced by amino acids with close or similar properties to form a polypeptide. These conservative variant polypeptides are best produced according to Table A by performing amino acid substitutions.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides analogs of the fusion protein of the present invention. The difference between these analogs and the polypeptide of the present invention may be a difference in amino acid sequence, may also be a difference in modified form that does not affect the sequence, or both. Analogs also include analogs having residues different from natural L-amino acids (such as D-amino acids), and analogs having non-naturally occurring or synthetic amino acids (such as β, γ-amino acids). It should be understood that the polypeptide of the present invention is not limited to the representative polypeptides exemplified above.

In addition, the fusion protein of the present invention can also be modified. Modified (usually without changing the primary structure) forms include: chemically derivative forms of polypeptides in vivo or in vitro, such as acetylation or carboxylation. Modifications also include glycosylation, such as those polypeptides produced by glycosylation modifications during the synthesis and processing of the polypeptide or during further processing steps. This modification can be accomplished by exposing the polypeptide to an enzyme that performs glycosylation (such as a mammalian glycosylase or deglycosylase). Modified forms also include sequences with phosphorylated amino acid residues (such as phosphotyrosine, phosphoserine, phosphothreonine). It also includes polypeptides that have been modified to improve their anti-proteolytic properties or optimize their solubility properties.

The term "polynucleotide encoding the fusion protein of the present invention" may include a polynucleotide encoding the fusion protein of the present invention, or a polynucleotide that also includes additional coding and/or non-coding sequences.

The present invention also relates to variants of the above-mentioned polynucleotides, which encode fragments, analogs and derivatives of polypeptides or fusion proteins having the same amino acid sequence as the present invention. These nucleotide variants include substitution variants, deletion variants and insertion variants. As known in the art, an allelic variant is an alternative form of polynucleotide. It may be a substitution, deletion or insertion of one or more nucleotides, but will not substantially change the function of the encoded fusion protein.

The present invention also relates to polynucleotides that hybridize with the aforementioned sequences and have at least 50%, preferably at least 70%, and more preferably at least 80% identity between the two sequences. The present invention particularly relates to polynucleotides that can hybridize with the polynucleotide of the present invention under strict conditions (or stringent conditions). In the present invention, "strict conditions" refer to: (1) hybridization and elution at lower ionic strength and higher temperature, such as 0.2×SSC, 0.1% SDS, 60°C; or (2) adding denaturant during hybridization, such as 50% (v/v) formamide, 0.1% calf serum/0.1% Ficoll, 42°C, etc.; or (3) hybridization occurs only when the identity between the two sequences is at least 90% or more, and more preferably 95% or more.

The fusion protein and polynucleotides of the present invention are preferably provided in an isolated form, and more preferably, are purified to homogeneity.

The full-length sequence of the polynucleotide of the present invention can usually be obtained by PCR amplification method, recombination method or artificial synthesis method. For the PCR amplification method, primers can be designed according to the relevant nucleotide sequence disclosed in the present invention, especially the open reading frame sequence, and a commercially available cDNA library or a cDNA library prepared according to a conventional method known to those skilled in the art is used as a template to amplify the relevant sequence. When the sequence is long, it is often necessary to perform two or more PCR amplifications, and then each amplified fragments are spliced together in the correct order.

Once the relevant sequences are obtained, the relevant sequences can be obtained in large quantities by recombination method. It is usually cloned into a vector, then transferred into a cell, and then the relevant sequence is isolated from the host cell after proliferation by conventional methods.

In addition, the relevant sequences can also be synthesized by artificial synthesis, especially when the fragment length is short. Usually, by first synthesizing multiple small fragments, and then ligating to obtain very long fragments.

At present, the DNA sequence encoding the protein (or fragment or derivative thereof) of the present invention can be obtained completely through chemical synthesis. The DNA sequence can then be introduced into various existing DNA molecules (or such as vectors) and cells known in the art.

The method of using PCR technology to amplify DNA/RNA is preferably used to obtain the polynucleotide of the present invention. Especially when it is difficult to obtain full-length cDNA from the library, the RACE method (RACE-cDNA end rapid amplification method) can be preferably used, and the primers used for PCR can be appropriately selected according to the sequence information of the present invention disclosed herein, and can be synthesized by conventional methods. The amplified DNA/RNA fragments can be separated and purified by conventional methods such as gel electrophoresis.

### Expression Vector

The present invention also relates to a vector containing the polynucleotide of the present invention, a host cell produced by genetic engineering using the vector of the present invention or the sequence encoding the fusion protein of the present invention, and a method for producing the polypeptide of the present invention through recombinant technology.

Through conventional recombinant DNA technology, the polynucleotide sequence of the present invention can be used to express or produce recombinant fusion protein. Generally, there are the following steps:
(1). using the polynucleotide (or variant) of the present invention encoding the fusion protein of the present invention, or using a recombinant expression vector containing the polynucleotide to transform or transduce a suitable host cell;
(2). culturing a host cell in a suitable medium;
(3). isolating and purifying protein from culture medium or cells.

In the present invention, the polynucleotide sequence encoding the fusion protein can be inserted into a recombinant expression vector. The term "recombinant expression vector" refers to bacterial plasmids, bacteriophages, yeast plasmids, plant cell viruses, and mammalian cell viruses such as adenovirus, retrovirus or other vectors well known in the art. Any plasmid and vector can be used as long as it can be replicated and stabilized in the host. An important feature of an expression vector is that it usually contains an origin of replication, a promoter, a marker gene, and translation control elements.

Methods well known to those skilled in the art can be used to construct an expression vector containing the DNA sequence encoding the fusion protein of the present invention and appropriate transcription/translation control signals. These methods include in vitro recombinant DNA technology, DNA synthesis technology, and in vivo recombination technology. The DNA sequence can be effectively linked to an appropriate promoter in the expression vector to guide mRNA synthesis. Representative examples of these promoters are: *Escherichia coli* lac or trp promoter; lambda phage PL promoter; eukaryotic promoters including CMV immediate early promoter, HSV thymidine kinase promoter, early and late SV40 promoter, retroviral LTRs and some other known promoters that can control gene expression in prokaryotic or eukaryotic cells or viruses. The expression vector also includes a ribosome binding site for translation initiation and a transcription terminator.

In addition, the expression vector preferably contains one or more selectable marker genes to provide phenotypic traits for selecting transformed host cells, such as dihydrofolate reductase, neomycin resistance, and green fluorescent protein (GFP) for eukaryotic cell culture, or tetracycline or ampicillin resistance for *E. coli.*

A vector containing the above-mentioned appropriate DNA sequence and an appropriate promoter or control sequence can be used to transform an appropriate host cell so that it can express the protein.

The host cell can be a prokaryotic cell, such as a bacterial cell; or a lower eukaryotic cell, such as a yeast cell; or a higher eukaryotic cell, such as a mammalian cell. Representative examples include: *Escherichia coli, Streptomyces;* bacterial cells of *Salmonella typhimurium;* fungal cells such as yeast and plant cells (such as ginseng cells).

When the polynucleotide of the present invention is expressed in higher eukaryotic cells, if an enhancer sequence is inserted into the vector, the transcription will be enhanced. Enhancers are cis-acting factors of DNA, usually about 10 to 300 base pairs, acting on promoters to enhance gene transcription. Examples include the 100 to 270 base pair SV40 enhancer on the late side of the replication initiation point, the polyoma enhancer on the late side of the replication initiation point, and adenovirus enhancers and the like.

Those of ordinary skill in the art know how to select appropriate vectors, promoters, enhancers and host cells.

Transformation of host cells with recombinant DNA can be carried out by conventional techniques well known to those skilled in the art. When the host is a prokaryote such as *Escherichia coli,* competent cells that can absorb DNA can be harvested after the exponential growth phase and treated with the CaCl2 method. The steps used are well known in the art. Another method is to use MgCl2. If necessary, transformation can also be carried out by electroporation. When the host is a eukaryote, the following DNA transfection methods can be selected: calcium phosphate co-precipitation method, conventional mechanical methods such as microinjection, electroporation, liposome packaging, etc.

The obtained transformants can be cultured by conventional methods to express the polypeptide encoded by the gene of the present invention. Depending on the host cell used, the medium used in the culture can be selected from various conventional mediums. The culture is carried out under conditions suitable for the growth of the host cell. After the host cells have grown to an appropriate cell density, the selected promoter is induced by a suitable method (such as temperature conversion or chemical induction), and the cells are then cultured for a period of time.

The antibody of the present invention can be expressed in the cell, on the cell membrane, or secreted out of the cell. If necessary, the physical, chemical, and other characteristics can be used to separate and purify the recombinant protein through various separation methods. These methods are well known to those skilled in the art. Examples of these methods include, but are not limited to: conventional renaturation treatment, treatment with protein precipitation agent (salting out method), centrifugation, bacteria broken through osmosis, ultra treatment, ultracentrifugation, molecular sieve chromatography (gel filtration), adsorption chromatography, ion exchange chromatography, high performance liquid chromatography (HPLC) and various other liquid chromatography techniques and combinations of these methods.

### Fmoc modification

The use of peptides is increasing in the field of biomedicine. Amino acids are the basic raw materials for the peptide synthesis technology. All amino acids contain α-amino and carboxyl groups, and some also contain side chain active groups such as: hydroxyl, amino, guanidyl and heterocyclic. Therefore, it is necessary to protect amino groups and side chain active groups in the peptide-connecting reaction, and remove the protective groups after synthesis of polypeptides, otherwise amino acid misconnection and many side reactions will occur.

Fluorenylmethoxycarbonyl (Fmoc) is a base-sensitive protective group that can be removed in concentrated ammonia or dioxane-methanol-4N NaOH (30:9:1) and 50% dichloromethane solutions of piperidine, ethanolamine, cyclohexylamine, 1,4-dioxane, pyrrolidone and other ammonias.

Under weakly alkaline conditions such as sodium carbonate or sodium bicarbonate, Fmoc-Cl or Fmoc-OSu is generally used to introduce Fmoc protective groups. Compared to Fmoc-Cl, Fmoc-OSu is easier to control reaction conditions and has fewer side effects. Fmoc has strong ultraviolet absorption, the maximum absorption wavelength is 267nm (ε18950), 290nm (ε5280), 301nm (ε6200). Thus it can be detected through ultraviolet absorption, which brings many conveniences to the automatic peptide synthesis by instruments. In addition, it can be compatible with a wide range of solvents and reagents, has high mechanical stability, and can be used with a variety of carriers and a variety of activation methods. Therefore, the Fmoc protection group is most commonly used in peptide synthesis now.

### Fmoc-OSu (9- -Fluorenyl methyl succinyl iminocarbonate)

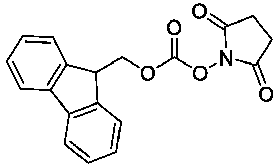

### Insulin degludec side chain

tBuO-Pal-Glu (OSu)-OtBu is the side chain of insulin degludec.

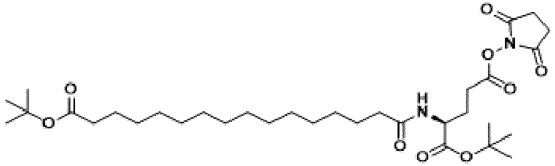

### tBuO-Pal-Glu(OSu)-OtBu

The preparation of insulin degludec is to first use genetic recombination technique to obtain the insulin degludec main chain with a Boc-protected lysine at position 29, and then connect the degludec insulin side chain tBuO-Pal-Glu(OSu)-OtBu to obtain insulin degludec.

### Production of insulin degludec

The synthesis route of insulin degludec provided by the present invention is as follow. Fmoc modified Compound 2 is produced from the Boc-insulin degludec main chain (Compound 1). Boc protection is removed from Compound 2 to obtain Compound 3. Compound 3 is reacted with activated insulin degludec side chain tBuO-Pal-Glu (OSu)-OtBu to obtain Compound 4. Then Compound 5 is obtained through Fmoc-removing reaction. OtBu protective group is removed from the side chain to finally obtain Compound 6 insulin degludec.

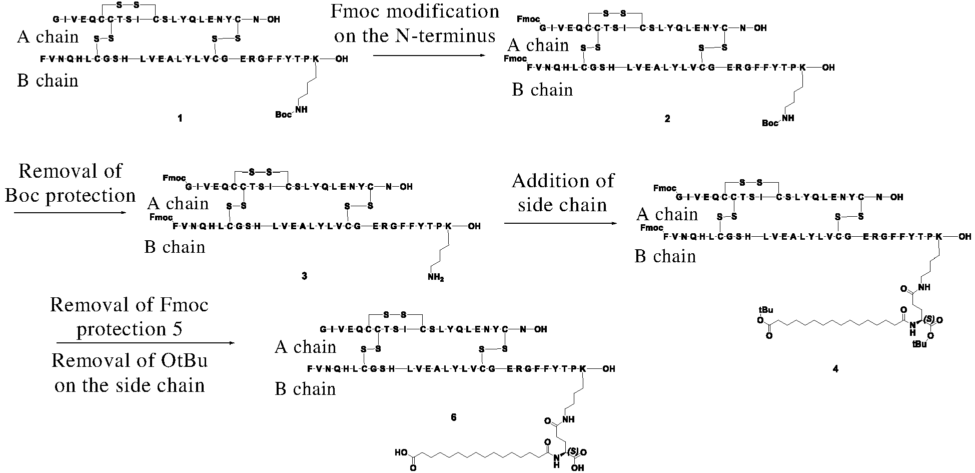

Specifically, the present invention provides a method for preparing insulin degludec, comprising the steps:
(i) providing a Boc-modified insulin degludec main chain;
(ii) modifying the Boc-modified insulin degludec main chain with Fmoc, thereby obtaining the Fmoc and Boc-modified insulin degludec main chain;
(iii) removing the Boc from the Fmoc and Boc-modified insulin degludec main chain, and reacting the same with an insulin degludec side chain, thereby obtaining a Fmoc-modified insulin degludec; and
(iv) removing the Fmoc from the Fmoc-modified insulin degludec and the OtBu from the side chain, thereby obtaining the insulin degludec.

### The present invention mainly has the following advantages:

(1) The present invention produces the Boc-modified insulin degludec main chain without adopting methods such as dilution, ultrafiltration and liquid replacement to remove excess inorganic salts in the supernatant of the fermentation broth. Secondly, there is no need for cyanogen bromide cleavage, oxidized sulfite hydrolysis and related purification steps in the preparation of the peptide of interest.
(2) In the method of the present invention, the fusion protein contains a high proportion of the insulin degludec main chain (the fusion ratio increases). The FP or A-FP in the fusion protein, which contain arginine and lysine, can be digested with proteases into small fragments whose molecular weight are quite different from the target protein, and can readily be separated. The one-step yield of separating Boc-insulin degludec main chain or similar precursor by using a chromatography column is more than 70%, which is 3 times higher than the conventional method, and can remove most of the pigment. The yield of the Boc-insulin degludec main chain is about 1.5-1.8 g/L. The synthesis process steps of the present invention are simplified by more than two-thirds, and the process time and equipment investment costs are reduced.
(3) Due to the Boc-lysine protection at position 29, the present invention can directly synthesize insulin degludec by orthogonal reaction with Fmoc protection.
(4) The insulin degludec synthesized through the method of the present invention has no N-terminal fatty acid acylation impurities, which is conducive to downstream purification and reduces costs.
(5) Compared with solid-phase synthesis, the method of the present invention does not produce racemic impurity polypeptides, and does not need to use a large number of modified amino acids, does not use a large number of organic reagents and has little environmental pollution and lower cost.

The present invention will be further elaborated below in conjunction with specific embodiments. It should be understood that these embodiments are only used to illustrate the present invention and not to limit the scope of the present invention. The experimental methods without specific conditions in the following examples are usually based on conventional conditions, or according to the conditions suggested by the manufacturer. Unless otherwise specified, percentages and parts are weight percentages and parts by weight.

### Example 1 Construction and expression of the insulin degludec expression bacterial strain

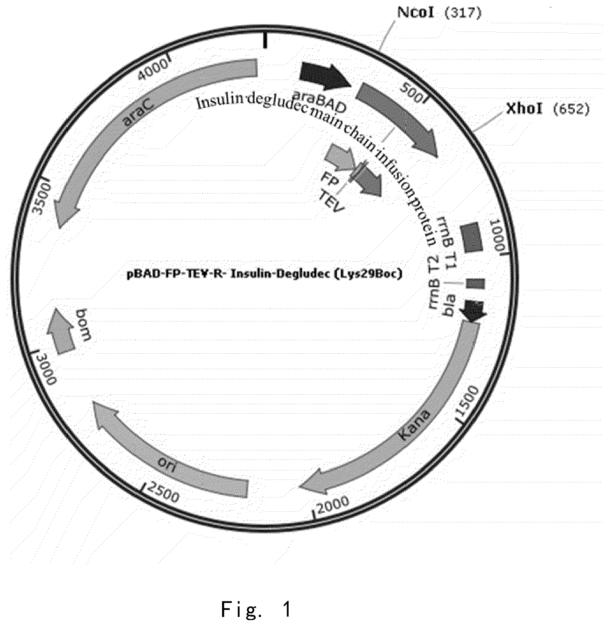

The construction of the insulin degludec expression vector refers to the description of Examples in the Chinese patent application No. 201910210102.9. The DNA fragment of the fusion protein pBAD-FP-TEV-R-Insulin (DesB₃₀, Lys²⁹Boc) was cloned to the NcoI-XhoI site downstream of the araBAD promoter of the expression vector plasmid pBAD/His A (purchased from NTCC, Kanamycin resistance) to obtain the plasmid pBAD-FP-TEV-R-Insulin (DesB₃₀, Lys²⁹Boc). The plasmid map is shown in Fig. 1.

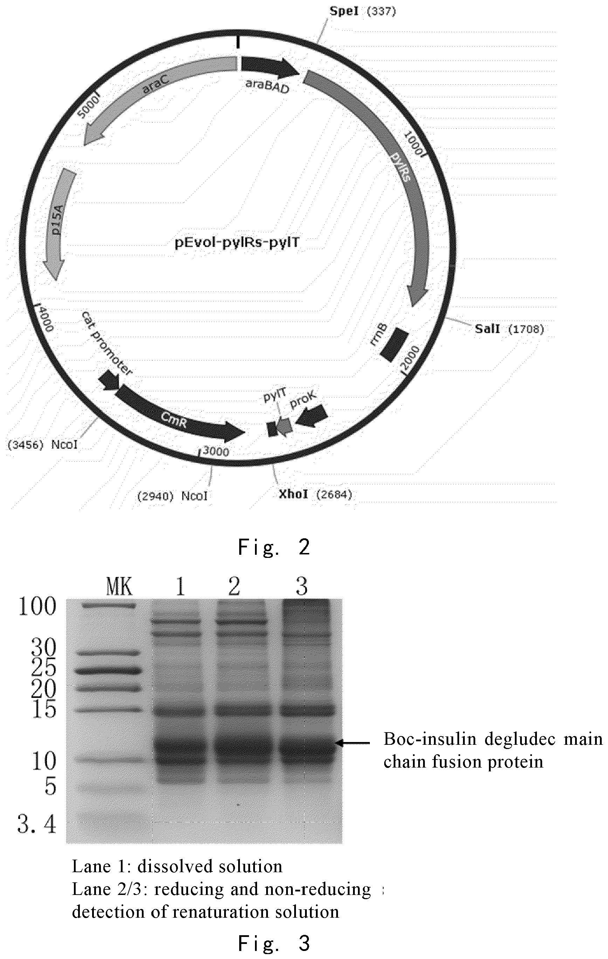

Then the DNA sequence of pylRs was cloned to the SpeI-SalI site downstream of the araBAR promoter of the expression vector plasmid pEvol-pBpF (purchased from NTCC, chloramphenicol resistance), and the DNA sequence of the tRNA (pylTcua) of lysyl-tRNA synthase was inserted downstream of the proK promoter by PCR. The plasmid is named as pEvol-pylRs-pylT. The plasmid map is shown in Fig. 2.

The constructed plasmid pBAD-FP-TEV-R-Insulin (DesB₃₀, Lys²⁹Boc) and pEvol-pylRs-pylT were co-transformed into *E. coli* TOP10 strains. The recombinant *E. coli* strains that express the insulin degludec main chain fusion protein FP-TEV-R-Insulin (DesB₃₀, Lys²⁹Boc) were screened and obtained.

MVSKGEELFTGVKLTLKFICTTYVQERTISFKDTYKTRAEVKFEGD
ENLYFQGRFVNQHLCGSHLVEALYLVCGERGFFYTPK(Boc)
RGIVEQCCTSICSLYQLENYCN (SEQ ID NO. 1)

Seed medium was prepared and inoculated, and a secondary seed solution was produced through two-stage cultivation. After 20 hours of cultivation, the OD₆₀₀ reached about 180 and the fermentation was terminated. About 3L of fermentation broth was obtained, and about 150g/L of wet bacteria was obtained by centrifugation. After centrifugation of the fermentation broth, the breaking buffer was added, and bacteria were broken twice by using a high-pressure homogenizer. After centrifugation, Tween 80 and EDTA-2Na were added and washed and then washed once with water, and the inclusion bodies were obtained by centrifugation and collection of the precipitate. About 40g wet weight of inclusion bodies could be finally obtained per liter of fermentation broth, and contained the insulin degludec precursor fusion protein in the inclusion bodies.

### Example 2 Renaturation and digestion of the Boc-degludec precursor inclusion body

7.5-9.0mol/L urea solution was added into the inclusion bodies obtained in Example 1 at a ratio of 1:10 by mass to volume (m/v), stirred and dissolved at room temperature. The total protein concentration of the inclusion body dissolved solution was controlled at 10-25mg/mL, pH is adjusted to 11.0-11.8 using NaOH, and 2-15mmol/L of β-mercaptoethanol was added and stirred evenly. The inclusion body dissolved solution was dropwise added to the renaturation buffer containing 0.2-1.0 mmol/L L-cystine, 5~20mmol/L sodium carbonate, 5∼20mmol/L glycine, 0.3∼1.0 mmol/L EDTA-2Na to dilute the inclusion body dissolved solution to 5-10 times and renature the same. The pH value of the fusion protein renaturation solution was maintained at 10.5-11.8, and the temperature was controlled at 4-8 °C. The renaturation time was 10-20h, and the renaturation rate was over 60% (see Fig. 3 for electrophoretic detection), and the insulin degludec main chain fusion protein containing a disulfide bond between the insulin degludec A chain and the insulin degludec B chain was obtained.

The renaturation solution of Boc-insulin degludec main chain fusion protein was taken, adjusted to pH 8.0-9.5, added with the recombinant trypsin at the ratio of 1:3000-1:10000 of recombinant trypsin and total protein of the renaturation solution, added with the recombinant carboxypeptidase B at the ratio of 1:5000-1:15000 of recombinant carboxypeptidase B and total protein of the renaturation solution, and digested at 15-25 °C for 20-40 hours to finally obtain the Boc-insulin degludec main chain. The digestion rate was higher than 70%.

### Example 3 Preliminary purification of the Boc-insulin degludec main chain

The insoluble mixture in the enzyme digestion solution was removed by ultrafiltration. According to the difference of protein isoelectric point, the Boc-insulin degludec main chain obtained in Example 2 was preliminarily purified by DEAE anion exchange chromatography to remove most of the impurities. The combined load between Boc-insulin degludec main chain and filler was controlled to be less than 50mg/mL. The Boc-insulin degludec main chain was collected through gradient elution. After crude purification, the purity of Boc-insulin degludec main chain was more than 70%, and the yield was more than 85%.

### Example 4 Reversed phase chromatography of Boc-insulin degludec main chain

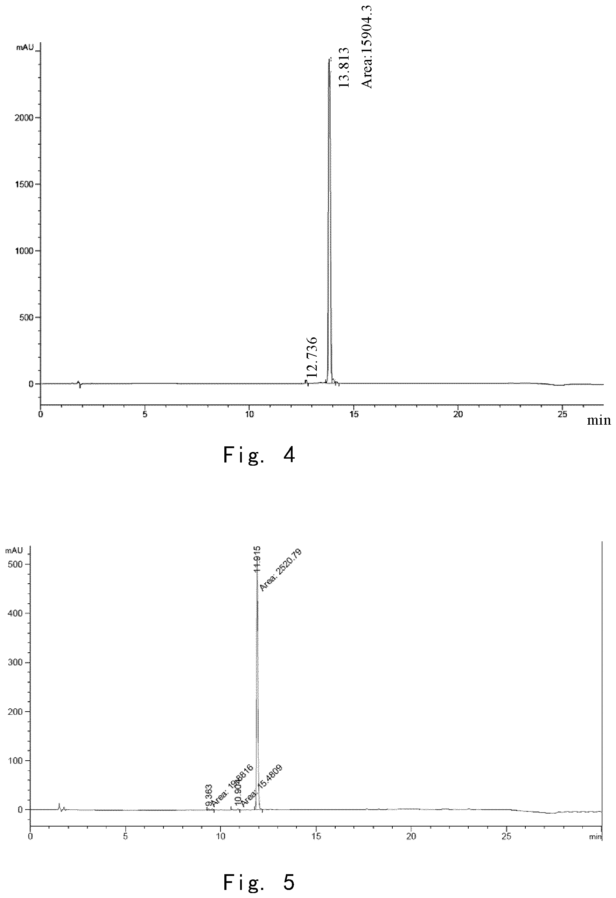

The Boc-insulin degludec main chain solution obtained in Example 3 was separated and purified by C8 reverse phase chromatography. The aqueous solution containing trifluoroacetic acid was used as mobile phase A; and acetonitrile solution containing trifluoroacetic acid was used as mobile phase B. The Boc-insulin degludec main chain was combined with C8 filler, the sample loading amount of Boc-insulin degludec main chain was controlled to be less than 10mg/mL, the gradient elution was performed, and the Boc-insulin degludec main chain was collected. The experimental result shows that the purity of the Boc-insulin degludec main chain collected by reverse phase chromatography was ≥ 90% (see Fig. 4 for HPLC detection diagram), and the yield was more than 60%.

### Example 5 Preparation of insulin degludec using Boc-insulin degludec main chain

The dried product of Boc-insulin degludec main chain Compound 1 obtained in Example 4 was taken(in this example, the molar ratio of the material is 30mg) and added with Fmoc-Osu, DIPEA and DMF according to the proportion in Table 1, and reacted for 8-12 hours to prepare the Fmoc and Boc-protected insulin degludec main chain. Then mixed solvent of methyl tert-butyl ether/petroleum ether (3:1) at 0 ± 5 °C was added to the reaction system. The solid precipitation was centrifuged, washed with the mixed solvent of methyl tert-butyl ether and petroleum ether (3:1) for 2-3 times to obtain Fmoc-protected Compound 2: DiFmoc Insulin (DesB₃₀, Lys²⁹Boc).

**Table 1 Molar ratio of materials**

| | Boc- insulin degludec main chain | Fmoc-OSu | DIPEA | DMF |
|---|---|---|---|---|
| Equivalent or volume | 1.0 eq | 5 eq | 12 eq | 1V |

Compound 2 was added to the precooled TFA solution at 0 ± 5 °C, stirred for 0.5-2.0h, and the cool mixed solution of methyl tert-butyl ether/petroleum ether (3:1) at 0 ± 5 °C was added to the reaction mixture, and then settled and centrifuged. The solid was washed with 30mg/ml methyl tert-butyl ether for 2-3 times and dried to obtain the Boc-removed solid Compound 3: DiFmoc-Insulin (DesB₃₀, Lys²⁹ NH₂).

Compound 3 after removal of Boc was taken and dissolved in DMF solution, added with 12 eq of DIPEA, and added with 2.5 eq of side chain compound tBuO-Pal-Glu(OSu)-OtBu at pH 8.0-9.0. The reaction mixture was gently stirred at room temperature for 2-3 hours. After reaction, mixed solvent of methyl tert-butyl ether/petroleum ether (3:1) at 0 ± 5 °C was added to precipitate the solid product, which was washed for 2-3 times and dried, and the white Compound 4: DiFmoc-Insulin (tBuO-Pal-Glu-(Lys²⁹NH)-OtBu, DesB₃₀) was obtained.

Compound 4 was taken and added into DMF solution containing 20% piperidine, and reacted at room temperature for 0.5-2.0 h. Then cool mixed solvent of methyl tert-butyl ether/petroleum ether (3:1) at 0 ± 5 °C was added to the reaction system to precipitate the product. The solid centrifugation was washed with methyl tert-butyl ether/petroleum ether (3:1) for 3-5 times and dried to obtain the Fmoc-removed Compound 5: Insulin (tBuO-Pal-Glu- (Lys²⁹NH)-OtBu, DesB₃₀).

Compound 5 after removal of Fmoc was taken and added into a mixed solution of TFA (trifluoroacetic acid), TIS (triisopropylsilane) and DCM (dichloromethane) ((90% TFA: 10% TIS): DCM=1:2), and shaking reacted at room temperature for 2-4 hours to remove the OtBu protective group on side chain. 10-20 times of the volume of cool mixed solvent of methyl tert-butyl ether/petroleum ether (3:1) at 0 ± 5 °C was added to the reaction system to precipitate and centrifuge. The solid was washed with mixed solvent of methyl tert-butyl ether/petroleum ether (3:1) for more than 3 times and pumped to finally obtain the insulin degludec.

After synthesis, the insulin degludec was subjected to two steps of C8 high pressure reverse phase chromatography to finally obtain insulin degludec with a purity of more than 99% (see Fig. 5).

### Comparative Example

The construction and expression of the fusion protein expression strain was carried out by using a method similar to that in Example 1, wherein the difference was merely that the amino acid sequence of the fusion protein used for expression is set forth in SEQ ID NO. 22.

MKKLLFAIPLVVPFYSHSTMELEICSWYHMGIRSFLEQKLISEEDLNSA
VDRFVNQHLCGSHLVEALYLVCGERGFFYTPK(Boc)RGIVEQCCTSICSLYQL
ENYCN (SEQ ID NO. 22)

The above fusion protein contains the B chain and A chain of insulin degludec, as well as a gIII signal peptide.

The results show that after 20 h of culture, OD₆₀₀ reached about 140 and the fermentation was terminated. About 3L of fermentation broth was obtained, and about 105g/L of wet bacteria was obtained by centrifugation. After centrifugation of the fermentation broth, the breaking buffer was added, and bacteria were broken twice by using a high-pressure homogenizer. The inclusion bodies were obtained by centrifugation and collection of the precipitate. About 30 g wet weight of inclusion bodies could be finally obtained per liter of fermentation broth, and contained in the inclusion body was the insulin degludec fusion protein.

The above results show that, compared with the expression of conventional structural fusion protein, the expression amount of the fusion protein of the invention is significantly increased, and the insulin degludec protein in the fusion protein is folded correctly, and has biological activity.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents are equally within the scope of the claims appended to this application.

## Claims

1. An insulin degludec precursor fusion protein having the structure as set forth in Formula III from N-terminus to the C-terminus:
A-FP-TEV-R-G (III)
wherein,
"-" represents a peptide bond;
A is absent or a leader peptide,
FP is a green fluorescent protein folding unit,
TEV is a first restriction site, and preferably is a restriction site of TEV enzyme;
R is arginine or lysine for enzyme digestion;
G is an insulin degludec major chain or an active fragment thereof; wherein the green fluorescent protein folding unit comprises 2 to 6 β-folding units selected from the group consisting of:
| β-folding unit | Amino acid sequence |
|---|---|
| u1 | VPILVELDGDVNG (SEQ ID NO. 11) |
| u2 | HKFSVRGEGEGDAT (SEQ ID NO. 12) |
| u3 | KLTLKFICTT (SEQ ID NO. 13) |
| u4 | YVQERTISFKD (SEQ ID NO. 14) |
| u5 | TYKTRAEVKFEGD (SEQ ID NO. 15) |
| u6 | TLVNRIELKGIDF (SEQ ID NO. 16) |
| u7 | HNVYITADKQ (SEQ ID NO. 17) |
| u8 | GIKANFKIRHNVED (SEQ ID NO. 18) |
| u9 | VQLADHYQQNTPIG (SEQ ID NO. 19) |
| u10 | HYLSTQSVLSKD (SEQ ID NO. 20) |
| u11 | HMVLLEFVTAAGI (SEQ ID NO. 21). |

2. The fusion protein according to claim 1, wherein the green fluorescent protein folding unit is u2-u3, u4-u5, u1-u2-u3, u3-u4-u5 or u4-u5-u6.

3. The fusion protein according to claim 1, wherein the G is a Boc-modified insulin degludec precursor which has the structure as set forth in Formula IV:
GB-X-GA (IV)
wherein,
"-" represents a peptide bond;
GB is the B chain of insulin degludec modified with Boc at position 29, whose amino acid sequence is set forth in SEQ ID NO. 2,
X is a linker peptide;
GA is the A chain of insulin degludec whose amino acid sequence is set forth in SEQ ID NO. 3.

4. The fusion protein according to claim 1, wherein the amino acid sequence of the insulin degludec precursor fusion protein is set forth in SEQ ID NO. 1.

5. An insulin degludec main chain fusion protein having the structure as set forth in Formula I from N-terminus to the C-terminus:
A-FP-TEV-R-D (I)
wherein,
"-" represents a peptide bond;
A is absent or a leader peptide,
FP is a green fluorescent protein folding unit;
TEV is a first restriction site, and preferably is a restriction site of TEV enzyme;
R is arginine or lysine used for enzyme digestion;
D is a Boc-modified insulin degludec main chain having the structure as set forth in Formula II: 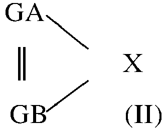 wherein,
"∥" represents a disulfide bond;
GA is the A chain of insulin degludec whose amino acid sequence is set forth in SEQ ID NO. 3,
X is a linker peptide;
GB is the B chain of insulin degludec modified with Boc at position 29, whose amino acid sequence is set forth in SEQ ID NO. 2;
wherein the green fluorescent protein folding unit comprises 2 to 6 β-folding units selected from the group consisting of:
| β-folding unit | Amino acid sequence |
|---|---|
| u1 | VPILVELDGDVNG (SEQ ID NO. 11) |
| u2 | HKFSVRGEGEGDAT (SEQ ID NO. 12) |
| u3 | KLTLKFICTT (SEQ ID NO. 13) |
| u4 | YVQERTISFKD (SEQ ID NO. 14) |
| u5 | TYKTRAEVKFEGD (SEQ ID NO. 15) |
| u6 | TLVNRIELKGIDF (SEQ ID NO. 16) |
| u7 | HNVYITADKQ (SEQ ID NO. 17) |
| u8 | GIKANFKIRHNVED (SEQ ID NO. 18) |
| u9 | VQLADHYQQNTPIG (SEQ ID NO. 19) |
| u10 | HYLSTQSVLSKD (SEQ ID NO. 20) |
| u11 | HMVLLEFVTAAGI (SEQ ID NO. 21). |

6. A Boc-modified insulin degludec precursor having the structure as set forth in Formula IV:
GB-X-GA (IV)
wherein,
"-" represents a peptide bond;
GB is the B chain of insulin degludec modified with Boc at position 29, whose amino acid sequence is set forth in SEQ ID NO. 2,
X is a linker peptide, and preferably the amino acid sequence of the linker peptide is R, RR, RRR or set forth in SEQ ID NO. 4-7; and
GA is the A chain of insulin degludec whose amino acid sequence is set forth in SEQ ID NO. 3.

7. A Boc-modified insulin degludec main chain having the structure as set forth in Formula II: 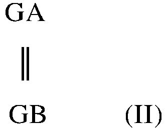 wherein,
" ∥" represents a disulfide bond;
GA is the A chain of insulin degludec whose amino acid sequence is set forth in SEQ ID NO. 3, and
GB is the B chain of insulin degludec whose amino acid sequence is set forth in SEQ ID NO. 2, and the lysine at position 29 of the B chain is an Nε-(tert-butoxycarbonyl)-lysine.

8. The main chain according to claim 7, wherein the N-terminus of both A chain and B chain are modified with Fmoc.

9. A Fmoc-modified insulin degludec main chain having the structure as set forth in Formula II: 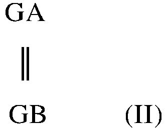 wherein,
"∥" represents a disulfide bond;
GA is the A chain of insulin degludec, whose amino acid sequence is set forth in SEQ ID NO. 3,
GB is the B chain of insulin degludec, whose amino acid sequence is set forth in SEQ ID NO. 2,
and the N-terminus of both A chain and B chain are modified with Fmoc.

10. An isolated polynucleotide, wherein the isolated polynucleotide encoding the insulin degludec precursor fusion protein of claim 1, the insulin degludec main chain fusion protein of claim 5, the insulin degludec precursor of claim 6, or the insulin degludec main chain of claim 7 or 8.

11. A method for preparing an insulin degludec, comprising the steps:
(A) using recombinant bacteria to ferment to prepare the insulin degludec precursor fusion protein of claim 1, and
(B) using the insulin degludec precursor fusion protein to prepare insulin degludec.

12. The method according to claim 11, wherein the step (B) further comprising the steps:
(ia) isolating and obtaining inclusion bodies of the insulin degludec precursor fusion protein from the fermentation broth of the recombinant bacteria, renaturing the inclusion bodies and obtaining the insulin degludec main chain fusion protein;
(ib) digesting the insulin degludec main chain fusion protein with enzyme, thereby obtaining a Boc-modified insulin degludec main chain;
(ii) modifying the Boc-modified insulin degludec main chain with Fmoc, thereby obtaining the Fmoc and Boc-modified insulin degludec main chain;
(iii) removing the Boc from the Fmoc and Boc-modified insulin degludec main chain, thereby obtaining a Boc-removed insulin degludec main chain;
(iv) reacting the Boc-removed insulin degludec main chain with an insulin degludec side chain, thereby obtaining a Fmoc-modified insulin degludec; and
(v) removing the Fmoc from the Fmoc-modified insulin degludec and the OtBu from the side chain, thereby obtaining the insulin degludec.

13. The method according to claim 12, wherein in the step (ii), Fmoc-Osu, DIPEA (N, N-diisopropylethylamine) and DMF (N, N-dimethylformamide) are added to carry out Fmoc modification.

14. The method according to claim 12, wherein the step (iii) further comprising the steps:
(a) adding the Fmoc and Boc-modified insulin degludec main chain to pre-cooled TFA (trifluoroacetic acid) solution, stirring at low temperature to remove Boc and obtain a Boc-removed product;
(b) purifying the Boc-removed product, preferably by using a mixture of methyl tert-butyl ether/petroleum ether, thereby obtaining the Boc-removed insulin degludec main chain.

15. A Fmoc-modified insulin degludec main chain having the structure as set forth in Formula II: 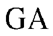 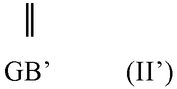 wherein,
"∥" represents a disulfide bond;
GA is the A chain of insulin degludec whose amino acid sequence is set forth in SEQ ID NO. 3,
GB' is the B chain of insulin degludec whose amino acid sequence is set forth in SEQ ID NO. 2, and the lysine at position 29 of the B chain is connected to a side chain of insulin degludec;
and the N-terminus of both A chain and B chain are modified with Fmoc.
